# DEMANDE DE BREVET EUROPEEN

(11) **EP 4 564 362 A1**
(43) Date de publication de la demande: **04.06.2025**
(21) Numéro de dépôt: 24215495.3
(22) Date de dépôt: 26.11.2024
(51) Int. Cl.: G16H 20/40, G16H 50/50, A61B 34/10, A61F 2/30, A61F 2/40

(54) **PROCÉDÉ DE SIMULATION DE L'ARTICULATION D'UN PATIENT**

(30) Priorité: 30.11.2023 FR 2313379
(71) Demandeur: FX Shoulder Solutions, 01440 Viriat (FR)
(72) Inventeur: DAUDET, Jean-Marie, 01440 Viriat (FR); MARTIN, Baptiste, 01440 Viriat (FR)
(74) Mandataire: LLR

(57) **Abrégé**

Procédé de simulation d'une articulation d'un patient en vue de planifier une opération chirurgicale d'implantation d'une prothèse articulaire 20, consistant à
- exploiter une image 1 de l'articulation du patient pour en tirer des informations géométriques sur la géométrie des os 3, et d'une partie au moins de tissus mous 2 la constituant ;
- à partir de ces informations, attribuer aux os des propriétés géométriques ;
- à partir de ces informations, attribuer aux tissus mous des points de liaison sur les os, ainsi que des forces de liaison estimées à partir des dimensions de ces tissus ;
- modéliser numériquement l'articulation en tenant compte des propriétés géométriques des os et des points de liaison des tissus mous, ainsi que des forces maximales de liaison des tissus mous ;
- intégrer une simulation numérique de la prothèse articulaire 20 au modèle numérique ;
- animer le modèle numérique obtenu après intégration de la prothèse, pour anticiper l'effet de celle-ci sur le patient opéré.

## Description

L'invention concerne le domaine des implants de prothèse articulaire.

Plus spécifiquement, l'invention concerne un procédé de simulation de l'articulation d'un patient en vue de planifier une opération chirurgicale d'implant de prothèse articulaire, par exemple une prothèse d'épaule.

Il est déjà connu, dans l'état la technique, de réaliser un modèle personnalisé de partie de squelette d'un patient devant recevoir une prothèse articulaire à partir d'un scanner réalisé au niveau de l'articulation ciblée. Le modèle permet de déterminer la géométrie de l'articulation et de choisir le modèle de prothèse le plus susceptible de restaurer les capacités de mouvement du patient une fois opéré. Les mouvements autorisés dépendent de la géométrie du squelette et des liaisons mécaniques mises en place par la prothèse.

Une difficulté du procédé de l'état de la technique et que, malgré les précautions prises par des préparatifs opératoires précis, on constate que les patients ne bénéficient pas tous identiquement des bienfaits de prothèses dont les modèles sont pourtant identiques. Il existe donc d'autres facteurs qui influencent le résultat de l'implantation.

Il a été avancé en première hypothèse que des maladies propres à chaque patient peuvent créer des différences qui influencent les résultats de l'implantation de la prothèse.

L'inventeur a émis et vérifié une hypothèse supplémentaire pour expliquer cette disparité de résultats entre les patients. Les tissus mous tels que ligaments, tendons, muscles, et le cartilage, qui ne sont pas pris en compte dans la simulation, ont une influence significative sur la réussite de l'implantation. S'ils étaient pris en compte, la simulation aboutirait à des choix potentiellement différents du modèle de prothèse et d'implantation de la prothèse sur le squelette, pour un résultat plus uniforme d'un patient à l'autre. Ce résultat s'est avéré exact à l'issue des tests réalisés par l'inventeur.

Dans la présente description, on désigne par « tissus péri-osseux » l'ensemble des tissus mous et du cartilage qui se trouvent en contact avec les os d'une articulation.

A cet effet, l'invention a pour objet un procédé de simulation d'une articulation d'un patient en vue de planifier une opération chirurgicale d'implantation d'une prothèse articulaire, le procédé consistant à :
- obtenir une image de l'articulation contenant des nuages de points caractéristiques de la géométrie non seulement des os du patient formant l'articulation, mais aussi d'une partie au moins des tissus péri-osseux constituant ladite articulation, lesdits tissus péri-osseux étant constitués par des tissus mous et/ou du cartilage qui se trouvent en contact avec les os du patient formant l'articulation,
- soumettre l'image contenant les nuages de points à une reconnaissance de formes, par comparaison avec une banque de données de parties anatomiques comprenant des informations relatives aux points d'insertion sur les os et aux forces de liaison des muscles, et/ou, en utilisant un apprentissage profond (en anglais : deep learning) et une intelligence artificielle, pour :
   ∘ identifier les os et les tissus péri-osseux constituant l'articulation, et
   ∘ leur attribuer des propriétés relatives aux points d'insertion sur les os et aux forces de liaison,
- créer un modèle numérique dit « segmenté » de l'articulation en tenant compte des os et des tissus péri-osseux identifiés et de leurs propriétés ainsi obtenues,
- intégrer une simulation numérique de prothèse articulaire au modèle numérique segmenté obtenu lors de l'étape précédente, en remplaçant les parties anatomiques à remplacer par la prothèse,
- animer le modèle numérique obtenu après intégration, pour anticiper l'effet de la présence de ladite prothèse sur le patient opéré.

Avantageusement, les tissus péri-osseux comprennent l'un au moins de l'ensemble constitué par les muscles, les tendons, les ligaments, le cartilage.

Dans un premier mode de réalisation de l'invention, l'image est obtenue par scanner, puis traitement d'image mettant en évidence les tissus mous qui ne sont normalement pas facilement visibles sur une image brute de scanner.

Selon un autre mode de réalisation, l'image est obtenue par IRM (imagerie par résonance magnétique).

Conformément à l'invention, selon un premier modèle, la modélisation numérique réalisée à partir de l'image consiste à repérer des points remarquables de l'image pour former des nuages de points et à construire des segments représentatifs des constituants du modèle. On appelle cette étape, consistant à transformer un nuage de points en solide, la segmentation.

Conformément à l'invention, selon un deuxième modèle on utilise une représentation par éléments finis. Chaque os et chaque tissu péri-osseux est représenté par un maillage de structures élémentaires.

Selon un mode de réalisation particulier de l'invention, dans le modèle numérique segmenté, chaque muscle est découpé en un nombre prédéterminé de fibres musculaires disjointes, chaque fibre musculaire recevant une valeur de force, qui est une composante de la force globale du muscle. Cette valeur de force peut varier dans le cycle du mouvement de l'articulation.

Selon un mode de réalisation particulier de l'invention, l'articulation est une épaule et les os sont l'humérus, le scapula et la clavicule.

Selon un autre mode de réalisation particulier, l'articulation est une hanche ou un genou.

Selon un mode de réalisation particulier de l'invention, les tissus péri-osseux pris en compte dans le modèle numérique segmenté sont les muscles de l'épaule, à savoir la coiffe des rotateurs, c'est-à-dire supra et infra spinatus, subscapularis et teres minor, et le deltoïde.

Conformément à l'invention, l'insertion des ligaments sur les os est prise en compte dans le modèle numérique segmenté de manière précise, ce qui permet de simuler très précisément les couples de forces exercées par les muscles sur les os.

Avantageusement, dans le modèle numérique segmenté, chaque muscle est indépendant des autres.

Avantageusement, dans le modèle numérique segmenté, chaque fibre d'un muscle est indépendante des autres fibres.

Dans un mode de réalisation particulier de l'invention, on simule une pathologie particulière du patient dans le modèle numérique segmenté en attribuant à chaque fibre d'un muscle et où à chaque muscle une valeur de force maximale dégradée.

Dans un mode de réalisation particulier de l'invention, l'animation du modèle numérique après intégration de la prothèse consiste à contracter numériquement chaque muscle ou fibre de muscle, c'est-à-dire à simuler l'application d'une force par chaque muscle ou fibre de muscle sur les os, et à observer le mouvement théorique des os dans leurs degrés de liberté laissés par la prothèse articulaire simulée.

L'invention permet donc de prévoir, de manière concrète et réaliste, les résultats futurs de l'implantation de la prothèse sur le patient.

Dans un mode de réalisation particulier, le procédé selon l'invention permet également de prédire quels muscles nécessiteront un développement avantageux pour le fonctionnement optimal de l'articulation. Ainsi, le praticien pourra recommander au patient des mouvements d'entraînement musculaire adaptés pour développer certains muscles qui procureront une meilleure efficacité de fonctionnement à l'articulation équipée de la prothèse. Un tel progrès n'était pas possible avec les modèles purement osseux de l'état de la technique.

L'invention permet que le chirurgien puisse planifier intégralement une intervention chirurgicale en tenant compte non seulement du squelette local du patient, mais également des tissus mous. Dans l'état de la technique la connaissance des tissus péri-osseux fait défaut et le chirurgien ne découvre qu'en début d'intervention chirurgicale quel est l'environnement du squelette du patient, constitué par les muscles, tendons, ligaments et cartilage. Le chirurgien doit donc s'adapter sur le vif, c'est-à-dire sans avoir pu s'y préparer, et éventuellement ajuster la fixation de l'articulation.

Conformément à l'invention, l'application de contractions sur des modèles de muscles du modèle numérique vise à obtenir des mouvements simples tel que : abduction adduction, flexion extension, rotation interne ou externe, circonvolutions.

Selon un mode de mise en oeuvre particulier de l'invention, l'animation du modèle numérique est renouvelée sous différentes hypothèses de développement musculaire du patient, de manière à anticiper le comportement de la prothèse non seulement dans le contexte de l'état de développement musculaire du patient avant l'opération, mais également dans des contextes évolutifs de développement musculaire du patient qui seraient recommandés par le praticien après l'opération.

Dans un mode de réalisation particulier, les informations issues de la banque de données comprennent la densité et/ou la qualité osseuse des os. Cette prise en compte permet d'améliorer la tenue dans le temps de la prothèse.

L'invention a également pour objet le modèle numérique du squelette local et des tissus péri-osseux environnant le squelette, obtenu par mise en oeuvre du procédé défini ci-dessus.

L'invention a également pour objet un procédé de sélection de prothèse à partir d'un choix de différentes prothèse disponibles, consistant à :
- tester successivement chacune des prothèses du choix de prothèses en implantant un modèle numérique de la prothèse et en animant le modèle numérique du squelette local et des tissus péri-osseux environnant le squelette intégrant ladite prothèse selon un champ de forces musculaires prédéterminé,
- comparer les résultats obtenus,
- une fois que tous les modèles de prothèse sont testés, sélectionner le modèle procurant les meilleurs résultats.

Dans un mode de mise en oeuvre particulièrement avancé, le procédé consiste à animer le modèle numérique avec plusieurs champs de forces musculaires, chaque champ de forces musculaires représentant un état de développement musculaire potentiel du patient, de manière à sélectionner la prothèse articulaire fournissant les meilleurs résultats dans un champ de force musculaire donné. Dans cette hypothèse, il sera recommandé au patient, après l'opération, de suivre un entraînement musculaire lui permettant de développer le champ de forces musculaires adapté à la prothèse ainsi sélectionnée.

L'invention a également pour objet un produit programme d'ordinateur permettant une planification chirurgicale, comprenant une série d'instructions permettant de mettre en oeuvre l'un au moins des procédés décrit ci-dessus.

### Brève description des figures

L'invention sera mieux comprise à la lecture de la description qui va suivre donnée uniquement à titre d'exemple et faite en se référant aux dessins annexés dans lesquels :

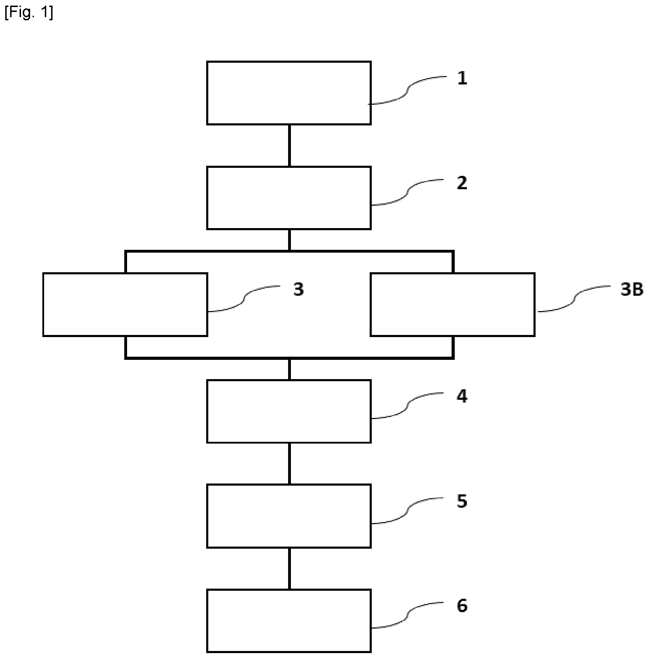

[Fig. 1] représente un organigramme illustrant un procédé selon l'invention
[Fig. 2] est une vue scannée en perspective et en coupe d'une épaule d'un patient, dans laquelle un implant scapulaire est représenté en vis-à-vis de la glène correspondante ;
[Fig. 3] est une vue similaire à celle de la figure 2, dans laquelle les tissus mous ont été retirés informatiquement et dans laquelle l'implant scapulaire est représenté implanté sur la scapula ;
[Fig. 4] est une vue illustrant un implant huméral, implanté dans une extrémité d'un humérus ; et,
[Fig. 5] est une vue similaire à celles des figures 2 et 3, dans laquelle les implants scapulaires et huméral sont représentés dans une position relative fonctionnelle.

### Description détaillée

Lors d'une première étape 1, on réalise une image d'une zone articulaire d'un patient, par exemple de son épaule. L'appareil d'imagerie utilisé est dans cet exemple un scanner.

Lors d'une deuxième étape 2, on soumet les images obtenues à un traitement d'images qui révèle davantage la géométrie des tissus péri-osseux entourant le squelette local du patient, de manière à mettre en évidence des nuages de points caractéristiques de ces tissus péri-osseux. Cette étape n'est pas nécessairement requise si l'image obtenue à l'étape 1 est suffisamment précise pour révéler immédiatement les nuages de points caractéristiques des os et des tissus péri-osseux.

On peut aussi utiliser une image combinant des données issues d'un scanner ou d'un appareil IRM.

Lors d'une étape suivante 3, on effectue une reconnaissance des éléments anatomiques présents sur l'image à l'aide d'une intelligence artificielle, qui compare les nuages de points de l'image à des représentations d'os et de tissus péri-osseux présents dans une banque de données contenant des informations géométriques sur les parties anatomiques et des informations sur les points d'insertion sur les os et sur les forces de liaison des muscles. On obtient ainsi un modèle numérique segmenté de l'articulation.

Dans une variante 3B de l'étape 3, chaque muscle est remplacé par dix fibres musculaires indépendantes, auxquelles on applique le même traitement. Dans ce cas, la contraction de chaque muscle se traduit par une contraction de chaque fibre, en tenant compte des propriétés individuelles de chaque fibre, dont la force, variable au cours du mouvement visé, peut avoir été ajustée manuellement pour tenir compte d'une maladie du patient.

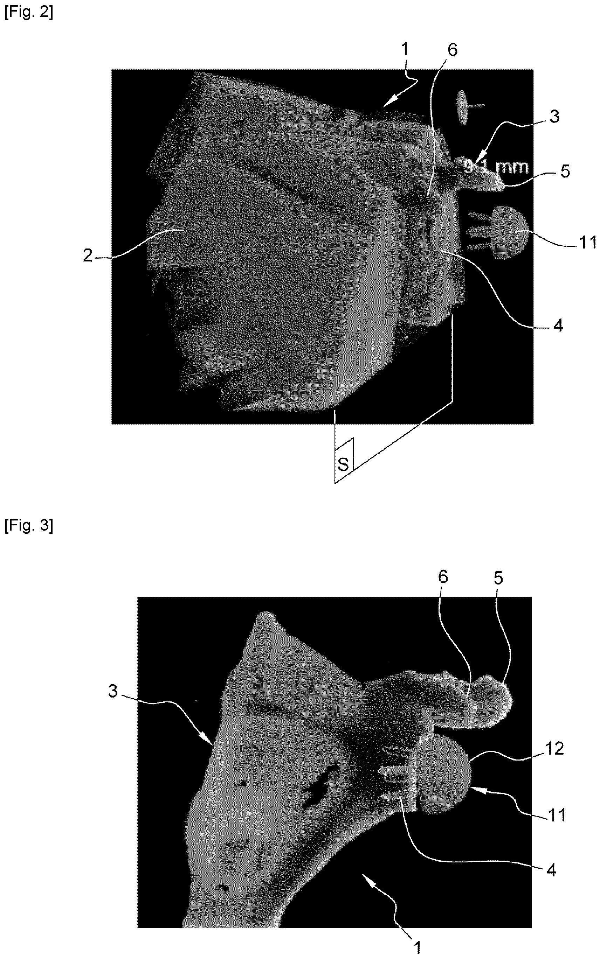

Lors d'une étape suivante 4, on sélectionne un modèle numérique d'une prothèse articulaire et on intègre ce modèle numérique dans le modèle numérique segmenté de l'articulation, en remplaçant numériquement les parties anatomiques à remplacer par la prothèse articulaire. Cette étape 4 est illustrée par les images des figures 2 à 5.

Lors d'une étape suivante 5, on anime le modèle numérique en appliquant des forces sur certains muscles pour simuler leurs contractions et l'on observe le mouvement ainsi produit sur le squelette lié par la prothèse articulaire.

Lors d'une étape suivante 6, on modifie l'implantation de la prothèse articulaire simulée à l'étape précédente jusqu'à obtention d'un comportement optimal de l'articulation. On entend par mouvement optimal un mouvement proche de celui d'un patient de morphologie similaire en bonne santé. Ainsi, on détermine le meilleur emplacement du ou des implants et la meilleure combinaison d'implants pour obtenir les meilleurs résultats. En outre, on détermine la musculation nécessaire que devra développer le patient après l'opération, pour optimiser sa récupération.

Le modèle d'éléments finis et le logiciel résultant de la mise en oeuvre du procédé qui vient d'être décrit peuvent être réalisés en mobilisant les connaissances générales de l'homme du métier et ne seront pas décrits ici.

Les figures 2 à 5 illustrent la pose selon l'invention d'une prothèse inversée. Dans l'exemple illustré, il s'agit de la pose d'une prothèse de l'articulation scapulo-humérale.

La figure 2 illustre une représentation numérique en trois dimensions de l'épaule gauche 1 du patient, dans laquelle les tissus mous, essentiellement des tissus musculaires 2 ont été informatiquement retirés à droite d'un plan de section S sensiblement vertical. On voit émerger de ces tissus mous 2, émergeant du plan de section S, des parties de la scapula 3, notamment : la glène 4, affleurante avec le plan de section S, l'acromion 5 et la coracoïde 6. La figure 2 illustre aussi un modèle numérique d'implant scapulaire 11, représenté à distance de la scapula 3. Par la suite, le modèle d'implant est simplement appelé « implant ».

Comme particulièrement illustré à la figure 3, l'implant scapulaire 11 est destiné à être implanté sur la scapula, à l'endroit de la glène 4.

A l'image 1 de la figure 3, les tissus mous ont été informatiquement entièrement retirés de la représentation de l'épaule, de sorte que l'on y voit seulement l'os de la scapula et l'implant scapulaire qui y est fixé.

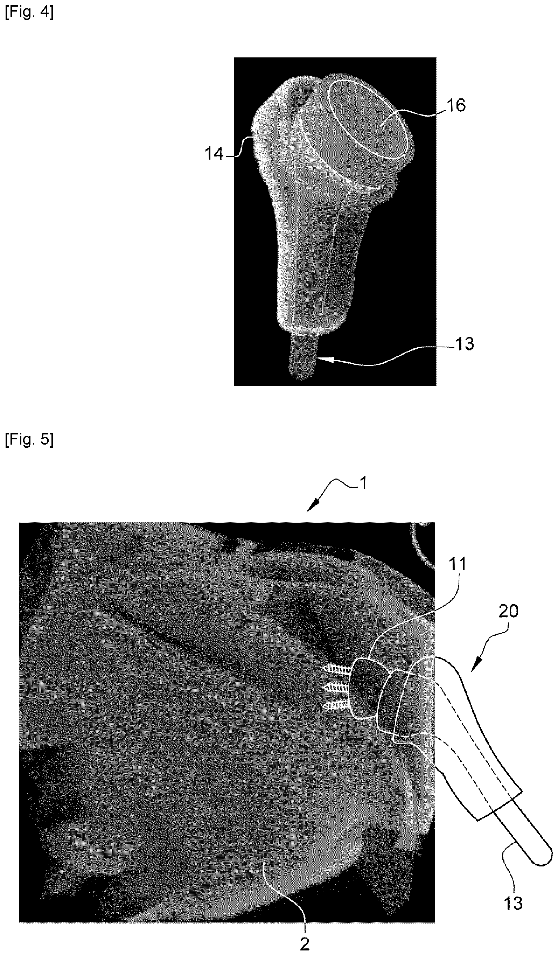

L'implant scapulaire remplace fonctionnellement la glène, sauf en ce qu'il comprend une surface articulaire 12 est de forme convexe. Ainsi, il est prévu pour former une prothèse inversée 20, avec un implant huméral 13 complémentaire dont un modèle numérique est visible aux figures 4 et 5.

La figure 4, illustre une représentation numérique en trois dimensions de la tête 14 de l'humérus gauche 1 du patient, sur laquelle les tissus mous ont été informatiquement retirés. Cette figure illustre aussi l'implantation de l'implant huméral 13, dans la tête 14 de l'humérus. L'implant 13 comprend une surface articulaire 16 concave, complémentaire à la surface articulaire 12 convexe de la glène. C'est le glissement des deux surfaces 12, 16 qui permet de fonctionnement de l'articulation 20.

La figure 5, est une représentation numérique de l'articulation 20, une fois réduite, dans la configuration et dans le contexte osseux et musculaire qu'elle est susceptible de prendre lors de son implantation réelle dans l'épaule du patient. L'image en trois dimensions de la figure 5 constitue donc un jumeau numérique de l'épaule du patient équipée de la prothèse, avant la pose de la prothèse.

Le jumeau numérique peut être anatomiquement déformé de façon à pouvoir modéliser un fonctionnement dynamique de la prothèse. Il peut être modifié, par exemple en changeant les formes et dimensions des implants, par exemple pour choisir ceux qui sont le mieux adaptés, s'insèrent le mieux dans l'anatomie et/ou permettent le plus grand confort d'usage au patient.

Ce jumeau numérique permet ainsi de programmer l'intervention. Il permet par exemple de modifier, en amont de l'intervention chirurgicale, la forme de l'implant scapulaire pour échapper à une interférence osseuse avec certaines parties de la scapula.

Ce procédé, grâce à la mise en place d'une prothèse mieux adaptée, en forme et en position, à l'anatomie du patient, permet une meilleure rééducation et une meilleure récupération. Il permet aussi de rendre une plus grande mobilité à l'articulation.

Bien sûr, l'invention n'est pas limitée aux exemples qui viennent d'être décrits. Au contraire, l'invention est définie par les revendications qui suivent.

Il apparaîtra en effet à l'homme de l'art que diverses modifications peuvent être apportées aux modes de réalisation décrits ci-dessus, à la lumière de l'enseignement qui vient de lui être divulgué.

Ainsi, le procédé précédemment décrit en référence à une prothèse inversée peut être utilisé pour une prothèse anatomique.

Aussi, le procédé peut être utilisé pour tout type de prothèse articulaire, pour laquelle il est important de vérifier la fonctionnalité de la prothèse dans chacune des positions qu'elle peut prendre, et aussi, dans son mouvement. Il peut aussi être utilisé pour des prothèse non mobiles pour vérifier et limiter son interférence avec le corps du patient.

## Revendications

1. Procédé de simulation d'une articulation d'un patient en vue de planifier une opération chirurgicale d'implantation d'une prothèse articulaire, le procédé consistant à
- obtenir une image (1) de l'articulation contenant des nuages de points caractéristiques de la géométrie non seulement des os (3, 14) du patient formant l'articulation, mais aussi d'une partie au moins des tissus péri-osseux constituant ladite articulation, lesdits tissus péri-osseux étant constitués par des tissus mous (2) et/ou du cartilage qui se trouvent en contact avec les os du patient formant l'articulation,
- soumettre l'image contenant les nuages de points à une reconnaissance de formes, par comparaison avec une banque de données de parties anatomiques comprenant des informations relatives aux points d'insertion sur les os et aux forces de liaison des muscles, et/ou, en utilisant un apprentissage profond et une intelligence artificielle, pour :
- identifier les os et les tissus péri-osseux constituant l'articulation ; et,
- leur attribuer des propriétés relatives aux points d'insertion sur les os et aux forces de liaison,
- créer un modèle numérique dit « segmenté » de l'articulation en tenant compte des os et des tissus péri-osseux identifiés et de leurs propriétés ainsi obtenues,
- intégrer une simulation numérique (20 ; 11, 13) de prothèse articulaire au modèle numérique segmenté obtenu lors de l'étape précédente, en remplaçant les parties anatomiques à remplacer par la prothèse,
- animer le modèle numérique obtenu après intégration, pour anticiper l'effet de la présence de ladite prothèse sur le patient opéré.

2. Procédé selon la revendication 1, dans lequel les tissus péri-osseux sont constitués de l'un au moins de l'ensemble constitué par les muscles, les tendons, les ligaments, le cartilage

3. Procédé selon l'une quelconque des revendications précédentes, dans lequel l'image est obtenue par scanner et/ou IRM, puis traitement d'image mettant en évidence les tissus mous.

4. Procédé selon l'une quelconque des revendications précédentes, dans lequel, dans le modèle numérique, chaque muscle est découpé en un nombre prédéterminé de fibres musculaires disjointes, chaque fibre musculaire recevant une valeur de force maximale, qui est une composante de la force maximale globale du muscle, qui peut varier durant un cycle d'animation dudit modèle.

5. Procédé selon l'une quelconque des revendications précédentes, **caractérisé en ce que** le modèle numérique réalisé à partir de l'image consiste à réaliser une segmentation, c'est à dire à repérer des points remarquables de l'image pour former des nuages de points et à construire des segments représentatifs des constituants du modèle.

6. Procédé selon la revendication 5, **caractérisé en ce que** chaque muscle est découpé en un nombre prédéterminé de fibres musculaires disjointes, chaque fibre musculaire recevant une valeur de force, qui est une composante de la force globale du muscle, ladite valeur de force pouvant varier dans le cycle d'animation du modèle.

7. Procédé selon l'une quelconque des revendications 1 à 4, **caractérisé en ce que** le modèle numérique réalisé à partir d'une représentation par éléments finis, chaque os et chaque tissu péri-osseux étant représenté par un maillage de structures élémentaires.

8. Procédé selon l'une quelconque des revendications précédentes, dans lequel l'articulation est une épaule et les os sont l'humérus, le scapula et la clavicule.

9. Procédé selon l'une quelconque des revendications précédentes, dans lequel les muscles sont ceux de l'épaule, à savoir la coiffe des rotateurs, c'est-à-dire supra et infra spinatus, subscapularis et teres minor, et le deltoïde.

10. Procédé selon l'une quelconque des revendications précédentes, dans lequel on simule une pathologie particulière du patient dans le modèle numérique en attribuant à chaque fibre d'un muscle et où à chaque muscle une valeur de force maximale dégradée.

11. Procédé selon l'une quelconque des revendications précédentes, dans lequel l'animation du modèle numérique après intégration de la prothèse consiste à contracter numériquement chaque muscle ou fibre de muscle, c'est-à-dire à simuler l'application d'une force par chaque muscle ou fibre de muscle sur les os, et à observer le mouvement théorique des os dans leurs degrés de liberté laissés par la prothèse articulaire simulée (20).

12. Modèle numérique servant à la planification d'une opération chirurgicale d'implantation d'une prothèse articulaire, obtenu par mise en oeuvre du procédé selon l'une quelconque des revendications précédentes.

13. Procédé de sélection de prothèse à partir d'un choix de différentes prothèse disponibles, consistant à :
- tester successivement chacune des prothèses du choix de prothèses en implantant un modèle numérique de la prothèse dans un modèle numérique selon la revendication 7 et en animant le modèle numérique intégrant ladite prothèse selon un champ de forces musculaires prédéterminé,
- comparer les résultats obtenus,
- une fois que tous les modèles de prothèse sont testés, sélectionner, de préférence automatiquement, de préférence en utilisant une intelligence artificielle, le modèle procurant les meilleurs résultats.

14. Procédé selon la revendication précédente, dans lequel on anime le modèle numérique avec plusieurs champs de forces musculaires, chaque champ de forces musculaires représentant un état de développement musculaire potentiel du patient, de manière à sélectionner la prothèse articulaire fournissant les meilleurs résultats dans un champ de force musculaire donné

15. Produit programme d'ordinateur permettant une planification chirurgicale, comprenant une série d'instructions permettant de mettre en oeuvre l'un au moins des procédés selon les revendications précédentes.
